# EUROPEAN PATENT APPLICATION

(11) **EP 1 731 176 A1**
(43) Date of publication of application: **13.12.2006**
(21) Application number: 05011786.0
(22) Date of filing: 01.06.2005
(51) Int. Cl.: A61L 15/44, A61L 27/34, A61L 27/54, A61L 31/10, A61L 31/16, C01B 21/24

(54) **Pre-treatment device comprising nitric oxide**

(71) Applicant: NOLabs AB, 252 21 Helsingborg (SE)
(72) Inventor: Peters, Tor, 8703 Erlenbach (CH)
(74) Representative: Asketorp, Göran

(57) **Abstract**

A device is provided that allows for pre-treatment of an area of a human or animal organ intended to be penetrated to connect the vascular system of said human or animal with a sampling, infusion, or withdrawal container. The device comprises nitric oxide (NO) for obtaining a vaso-dilating effect at said area during said pre-treatment.

## Description

### Field of the Invention

This invention pertains in general to the field of a device configured for preparing a subcutaneous tissue to insertion of a catheter, venflon®, needle and/or syringe. More particularly the invention relates to a device for preparing a subcutaneous tissue to insertion of a catheter, venflon®, needle, and/or syringe and a process for manufacturing of said device, involving the use of nitric oxide (NO).

### Background of the Invention

Catheters, venflons®, needles, and/or syringes are well known for being used to fluidly communicate with the vascular system of a patient in a minimally invasive procedure, whether to withdraw fluids from the patient or to infuse fluids into the patient.

venflons® and catheters are generally short thin flexible tubes that are open at a distal end and secured within a hub at a proximal end. The hub serves as a quick disconnectable mechanical connector between the venflon® or catheter and a delivery tube extending, for example, from a liquid source or a liquid withdrawal source.

Needles and syringes are unflexible, preferably made of a metallic material, devices with a tubular part, which are used to assist in application of catheters and venflons®, according to below, and direct sampling, infusion, and withdrawal of body fluids.

One typical catheter is an over-the needle style catheter that requires an insertion needle passing there through to penetrate the patient's skin and advance the catheter into the patient's vascular system. The needle comprises a bevelled distal end to facilitate piercing the patient's skin.

During the insertion of the devices according to above it is often very complicated for the nurse or physician to find a suitable vessel in connection to the vascular system of the patient. This complication is caused by low vaso-dilation in the subcutaneous tissue in the target area.

Also, in the field of this technology, nurses and physicians, normally disinfect an area that is to be penetrated by the venflon®, catheter, needle, or syringe. This is usually done by rubbing said area with a cotton pad supplied with some kind of alcohol.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This antipathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of a device for pretreatment of a subcutaneous area, that is to be penetrated by a venflon®, catheter, needle, or syringe, to increase vaso-dilation and simultaneously provide an antibacterial and ant-viral effect, by elution of nitric oxide NO).

Hence, an improved device for pretreatment of a subcutaneous area, that is to be penetrated by a venflon®, catheter, needle, or syringe, which device does increase circulation in said area, has a vaso-dilating effect, is easy to use, does not develop resistance against the active pharmaceutical substance, and provides anti-microbial and anti-viral effect, etc, would be advantageous, and in particular a device allowing for facilitating insertion of venflons®, catheters, needles, and syringes, etc., would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, at least partly by providing a device according to the appended patent claims.

According to one aspect of the invention, a device is provided that allows for pretreatment of an area of a human or animal organ, which organ is to be penetrated to connect the vascular system of said human or animal with a sampling, infusion, or withdrawal container. Said device comprises a nitric oxide (NO) eluting polymer arranged to contact said tissue, such that a therapeutic dose of nitric oxide is eluted from said nitric oxide eluting polymer to said tissue, allowing for increased vaso-dilation, and anti-microbial and anti-viral effect.

The organ according to the present invention may for example be the skin on the head, face, neck, shoulder, back, arm, hand, stomach, genital, thigh, leg, or foot, of a body of said human or animal.

According to another aspect of the invention, a manufacturing process for such a device is provided, wherein the process is a process for forming a device that allows for pre-treatment of an area of a human or animal organ, which organ is to be penetrated to connect the vascular system of said human or animal with a sampling, infusion, or withdrawal container. The process comprises selecting a plurality of nitric oxide eluting polymeric fibers, and deploying said nitric oxide eluting fibers in a patch/pad, dressing, tape/coating, plaster/sheath, gel, hydrogel, foam, cream, etc., to be comprised in said device.

The present invention has at least the advantage over the prior art that it provides target exposure of an organ area to NO, whereby an increased circulation in the organ area, a vaso-dilating effect, anti microbial and anti-viral effect, while not developing resistance against the active pharmaceutical substance, local skin irritation, pain etc, are simultaneously obtained.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a patch/pad according to the invention,
Fig. 2 is a schematic illustration of a tape or coating according to the invention, and
Fig. 3 is a schematic illustration of a sheath or plaster according to the invention.

### Description of Embodiments

The following description focuses on embodiments of the present invention applicable to a device, in form of a patch/pad, dressing, gel, hydrogel, foam, cream, tape/coating, etc., which allows for pretreatment of an area of a human or animal organ, which area is to be penetrated to connect the vascular system of said human or mammal with a sampling, infusion, or withdrawal container, as well as a manufacturing method for the latter and a use of nitric oxide. This sampling, infusion, or withdrawal container may for example be, or be in communication with, or connected to, a catheter, venflon®, syringe, or needle, but the sampling, infusion, or withdrawal container according to the present invention is not intended to be limited to these examples. These examples are only intended to be illustrative in respect of the present invention. The registered trademark venflon® is used in the present invention not to limit the scope of the present invention but merely to give an example of what devices are included, and all devices functioning in the same way as venflon® are also within the scope of the present invention.

The animal according to the present invention may for example be selected from any mammal, such as cat, dog, horse, cattle, bird, pig, etc., or any other possible animal with a vascular system.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, i.e. 100 to 1000 folded higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

The polymers employed in embodiments of the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers. Gas stream spinning is suited for producing devices according to certain embodiments of the invention.

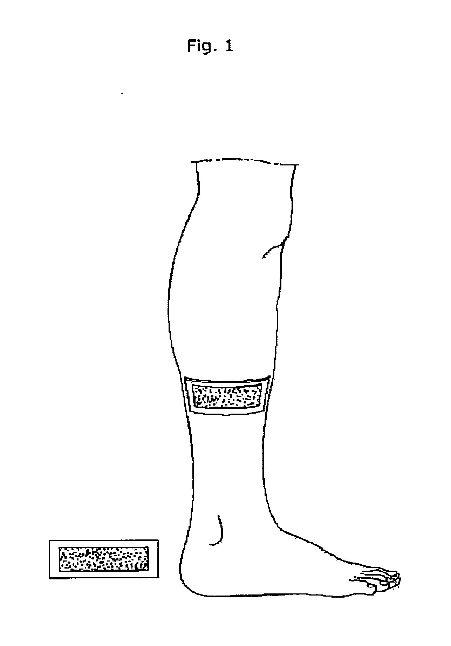

In an embodiment of the invention, according to Fig. 1, the device according to the present invention is in patch/pad, manufactured of a combination of L-PEI or other NO-eluting polymer and other suitable polymers, such as polyurethane, polyvinylacetates, polylacticacids, starch, cellulose, polyhydroxyalkanoates, polyesters, polycaprolactone, polyvinylalcohol, protein based plastics, gelatine, biogradable polymers, and latex, as base material, where NO is allowed to be eluted, said patch/pad being covered on the inside with nano-filament of NO-eluting L-PEI. The base material of the patch/pad according to the present invention may also be cotton, polyacrylate or any other fabric used in the clothing industry, in which cases the base material is loaded with the NO-eluting polymer according to the invention. This embodiment provides an easy to use patch/pad, which is applied on the area to be penetrated by a catheter, venflon®, syringe, or needle.

When the patch/pad according to an embodiment of the present invention gets in contact with the skin, and thereby gets in contact with the moisture in form of secreted sweat, the No-eluting patch/pad starts to release NO to said area.

The thus eluted NO has a vasodilating effect on the area, which effect results in that the blood vessels in said area will expand. Expanded blood vessels are easier to locate, which make it easier to the nurse or physician to choose which blood vessel to insert the catheter, venflon®, syringe, or needle, in. It is also much easier to the nurse or physician to penetrate the blood vessel with said catheter, venflon®, syringe, or needle, when the blood vessel is expanded.

NO has not only a vasodilating effect but also provides an anti-microbial.and anti-viral effect. Thus, there is no need to disinfect the area, intended to be subjected to insertion of a catheter, venflon®, syringe, or needle, with for example alcohol, which is common practice in the caretaking of today.

In another embodiment of the present invention the patch/pad is covered on the inside with nano-filament of any other suitable polymer, according to above. Such polymers are for example other polyalkyleneimines, such as B-PEI (Branched PolyEthyleneImine) or PEI-cellulose, which polymers have the advantage of being biocompatible, after the release of nitrogen oxide.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

Thus, it is most preferable that the nano-spun fibres in the No-eluting patch/pad according to the present embodiment of the present invention comprise PEI. Also nano-filaments to be woven into the patch/pad are suitably produced from PEI and loaded with NO for release thereof at use.

In another embodiment of the present invention the patch/pad according to the present invention is covered on the inside with NO-eluting nano-particles, or micro-spheres. These nano-particles, or micro-spheres, may be formed from the NO-eluting polymers according to the present invention, encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics. When the nano-particles, or micro-spheres, according to this embodiment, gets in contact with the secreted moisture, in form of sweat, on the inside of the patch/pad, they start to elute NO on the area to be pre-treated.

In yet another embodiment of the present invention the patch/pad contains a small water bag or sealed water sponge. This water bag or sealed water sponge is used to activate the elution of NO from the NO-eluting polymer, nano-particles, and/or micro-spheres. Persons that not easily sweat may be helped by the use of this embodiment. Alternatively, the patch/pad may be soaked with water after, or just before, it is applied on said area.

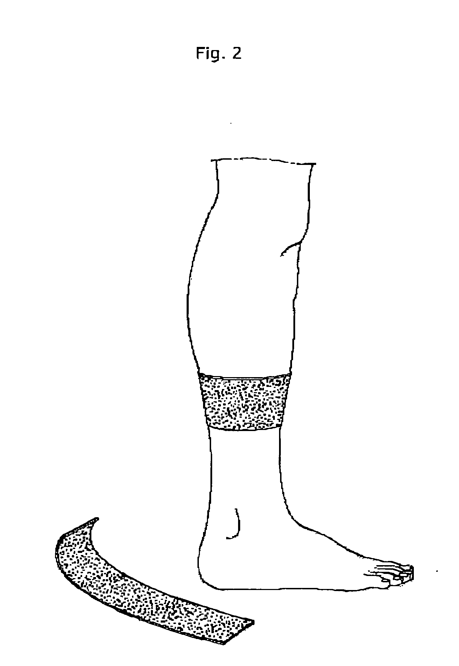

In still another embodiment of the device according to the present invention, it may be manufactured in the form of a polyurethane, or polyethylene, tape or coating, according to Fig. 2. This polyurethane tape or coating may easily be applied on the area intended to be subjected to insertion of a catheter, venflon®, syringe, or needle. At least the side facing the body part may be covered with NO-eluting nano-particles, micro-spheres, or nano-filament of NO-eluting L-PEI. when these particles or filaments get in contact with the moisture, in form of sweat, or water applied in any other way, on the inside of the tape or coating, the elution of NO starts.

This embodiment makes it possible to obtain a device that may be applied on locations that are difficult to get at with a patch/pad, such as in between the toes or fingers, the groin, the armpit etc.

In other embodiments of the invention, the tape/coating may be manufactured by any other suitable material, such as rubbers and plastics, polyvinylacetates, polylacticacids, starch, cellulose, polyhydroxyalkanoates, polyesters, polycaprolactone, polyvinylalcohol, protein based plastics, gelatine, biogradable polymers, and other soluble plastics, which material then is covered by an NO eluting polymer according to the present invention.

In another embodiment the nano-particles, or micro-spheres according to above, may be integrated in a soluble film that disintegrates on the inside of the patch/pad or tape/coating according to the present invention, in order to elute NO at the area of interest when the soluble film gets in contact with the moisture, in form of sweat or from the water bag or sealed water sponge, on the area to be treated.

When placed on an area to be pre-treated the device according to the present invention provides NO-elution, which results in vasodilating effect. This vasodilating effect expands the blood vessels, which expansion facilitate insertion of a catheter, venflon®, syringe, or needle in said blood vessel.

In another embodiment of the present invention the device only allows NO-elution in one direction. In this kind of embodiment one side of the patch/pad or tape/coating is non-permeable to NO. This may be accomplished by applying a material on one side of the patch/pad or tape/coating that is not permeable to NO. Such materials may be chosen from the group comprising common plastics, such as polyethylene, polyurethane, polyesters, polyamides, polyethers, polycarbonates, polyacrylonitrile, polystyrene, polypropylene, poly(acrylic acid) etc.

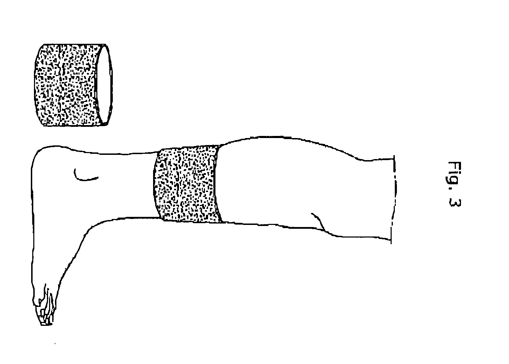

In another embodiment of the present invention, the device is in form of polyurethane or polyethylene sheaths or plasters, pads or dressings according to Fig. 3, coated with the NO-eluting polymer according to the present invention. The plaster or sheath may be applied on the area intended for insertion of a catheter, venflon®, syringe, or needle.

In other embodiments of the present invention the devices are covered with a powder, manufactured from nano-fibres of NO-eluting polymer, such as L-PEI, B-PEI, and/or PEI-cellulose. In this embodiments the devices according to the present invention are covered with said powder in the same way as the devices according to above were covered with nano-particles and/or micro-spheres.

In still another embodiment of the present invention the patch/pad, tape/coating, sheath/plaster, or dressing, according to above, is packaged in an air and/or light tight protective packaging. When one side of the protective packaging is removed a side covered with the NO eluting polymer according to the embodiments of the present invention is applied on the area to be pre-treated, on which area the device starts to elute NO.

In still another embodiment of the present invention the device is packaged in a protective packaging comprising a water bag, or other suitable water reservoir. Just before application of the device on the area to be pre-treated the water bag, or other suitable water reservoir, is broken. Thereafter the wetted device according to the present invention is applied on the area to be pre-treated, after which the device starts to elute NO.

In another embodiment of the device according to the present invention the fibres, nano-particles, micro-spheres, and/or powder may be integrated in a gel, that may either be in a smearing or compressed structure. The elution of NO may then be initiated by applying a water soaked patch on said gel. The fibres, nano-particles, or micro-spheres may also be integrated in a hydrogel, which is mixed directly before use. These embodiments have the advantage of being able to penetrate pockets and corners in the skin for closer elution of NO on the area to be pretreated.

In still another embodiment the device of the present invention is in form of a cream, spray or foam. The cream, spray or foam may comprise the NO eluting polymer in a non aqueous solvent, such as an oil based solvent. First, the cream, spray, or foam is applied on the area to be pretreated, then water, or another aqueous solution is applied to initiate the elution of NO. It is also possible to have a cream, spray, or foam comprising NO eluting polymer in a coating material according to above, which coating material breaks upon pressure, which breakage initiate elution of NO.

All embodiments of the present invention may be provided with an adhering material, such as a glue, etc., for facilitating the application of the devices on the area intended to be penetrated by the catheter, venflon®, syringe, needle, etc.

The device according to the present invention elutes nitric oxide (NO) from said eluting polymer in a non toxic dose, such as between 0.001 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The NO-eluting polymers in the devices according to the present invention may be combined with silver, such as hydroactivated silver. The integration of silver in the devices according to the present invention gives the anti-microbial and anti-viral effect an extra boost. Preferably the silver is releasable from the devices in the form of silver ions.

In yet another embodiment of the present invention the NO-eluting device is acting as a booster for drug eluting patches, e.g. pharmaceuticals, vitamins, nicotin, nitroglycerin, Non-Steroidal Anti.Inflammatory Drugs (NSAID), such as diclofenac, ibuprofen, aspirin, naproxen, COX-2 inhibitors, choline magnesium trisalicylate, diflunisal, salsalate, fenoprofen, flurbiprofen, ketoprofen, oxaprozin, indomethacin, sulindac, tolmetin, meloxicam, piroxicam, meclofenamate, mefenamic acid, nabumetone, etodalac, ketorolac, celecoxib, valdecoxib, and rofecoxib; steroids, such as cortisone, prednisone, methylprednisolone, prednisolone, vitamin D, estrogen, cholestrol, beclomethasone, flunisolide, fluticasone, triamcinolone, desonide, clobetasol, alclometasole, desoximetasone, betamethasone, halcinonide and dexamethasone; pain reliefs, such as motrin, feldene, naprosyn, lidocaine, and prilocaine; and other substances, such as indinavirsulfate, finasteride, aprepitant, montelukast sodium, alendronate sodium, rofecoxib, rizatriptan benzoate, simvastatin, finasteride, ezetimibe, caspofungin acetate, ertapenem sodium, dorzolamide hydrochloride, timolol maleate, losartan potassium, and hydrochlorotiazide; etc. This embodiment presents a device with the advantage of combining two treatments, of significant value, in one treatment.

The device according to the present invention may be manufactured by, for example electro spinning of for example L-PEI. L-PEI is then charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

The basic material of the device according to the present invention may be polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, gelatine, and other biocompatible polymers. The NO-eluting polymer may be integrated in, spun together with, or spun on top of, any of these materials in all of the embodiments of the present invention.

It is of course possible to electro spin the other NO-eluting polymers, according to above, on the device according to the invention while still being inside the scope of the present invention.

In one embodiment the NO-eluting polymers employed in the devices according to the present invention are electro spun in such way that pure NO-eluting polymer fibres may be obtained.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres or micro particles with the area to be pretreated, effective use of NO-eluting polymer, and a cost effective way of producing the device according to the present invention.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device configured for pre-treatment of an area of a human or animal organ intended to be penetrated to connect the vascular system of said human or animal with a sampling, infusion, or withdrawal container, wherein
said device comprises nitric oxide (NO) for obtaining a vaso-dilating effect at said area during said pretreatment.

2. The device according to claim 1, wherein said device comprises a polymer configured for eluting a non-toxic dosage of nitrogen oxide (NO) when used for said pretreatment.

3. The device according to claim 1, wherein said polymer is selected from the group consisting of branched and linear polyalkyleneimines, polyalkyleneimine celluloses, S-nitrosylated polymer, and poly(alkylenimine)diazeniumdiolates, or any combination of these.

4. The device according to claim 1, wherein said polymer is L-PEI (linear polyethyleneimine), loaded with nitric oxide(NO), arranged for release of the nitric oxide (NO) at said area on a body for pre-treatment of said area.

5. The device according to claim 1, wherein said device has a form selected from the group comprising of a patch/pad, a tape/coating, a dressing and a sheath/plaster, configured for pre-treatment of an area of a human or animal intended to be penetrated to connect the vascular system of said human or animal with a sampling, infusion, or withdrawal container.

6. The device according to claim 5, wherein said patch/pad, tape/coating, dressing, or sheath/plaster is manufactured from polyurethane or polyethylene, and said patch/pad, tape/coating, dressing, or sheath/plaster includes said nitric oxide (NO) eluting polymer configured for in use eluting said nitric oxide (NO) to said area.

7. Device according to any of claims 1 to 6, including a water bag or sealed water sponge.

8. Device according to claim 7, wherein the water bag or sealed water sponge is included in a protective packaging of said device.

9. Device according to claim 1, wherein said device is packaged in a protective packaging prior to use.

10. Device according to claim 1, wherein said device is partly disintegrable when subjected to moisture or water.

11. Device according to claim 1, wherein said polymer comprises silver, configured for exposure of said area.

12. Device according to claim 1, wherein said device is configured to act as a booster for other active ingredients chosen from the group consisting of pharmaceuticals, vitamins, nicotin, nitroglycerin, Non-Steroidal Anti-Inflammatory Drugs, steroids, and/or pain reliefs.

13. Device according to claim 1, wherein said polymer is in form of nano-particles or micro-spheres.

14. Device according to claim 13, wherein said nano-particles, or micro-spheres, are encapsulated in suitable material, such as polyvinylacetates, polylacticacids, starch, cellulose, polyhydroxyalkanoates, polyesters, polycaprolactone, polyvinylalcvhol, protein based plastics, and/or gelatine.

15. Device according to claims 14 and 14, wherein said nano-particles, or micro-spheres, are integrated in a gel, hydrogel, foam, spray, or cream.

16. Device according to claim 1, wherein said device comprises a material adapted to regulate or controlelution of said NO, selected from the group comprising of polyvinylacetates, polylacticacids, starch, cellulose, polyhydroxyalkanoates, polyesters, polycaprolactone, polyvinylalcohol, protein based plastics, gelatine, and/or biogradable polymers.

17. Device according to claim 1, wherein said NO-eluting polymer is applied on, or integrated with, a material selected from the group consisting of polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, and gelatine, or combinations thereof.

18. A manufacturing process for a device configured pre-treatment of an area of a human or animal organ intended to be penetrated to connect the vascular system of said mammal with a sampling, infusion, or withdrawal container according to claim 1, comprising:
selecting a plurality of nitric oxide eluting polymeric particles, preferably nano fibres, nano particles or micro spheres, and
deploying said nitric oxide eluting particles into a suitable form, or as a coating onto a carrier, to form said device,
wherein said deploying comprises electro, air, or gas stream spinning of said fibres.

19. Use of a nitric oxide (NO) eluting polymer for the manufacture of a device configured for pre-treatment of an area of a human or animal intended to be penetrated to connect the vascular system of said mammal with a sampling, infusion, or withdrawal container, wherein
nitric oxide is loaded to said device, which device elutes nitric oxide (NO) from said eluting polymer in a non-toxic dose when used on said area.

20. Use according to claim 18, wherein said non-toxic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
